(19) Europäisches Patentamt
European Patent Office
Office européen des brevets

(11) Veröffentlichungsnummer: **0 044 809**
**B1**

(12) # EUROPÄISCHE PATENTSCHRIFT

(45) Veröffentlichungstag der Patentschrift: **31.08.83**

(21) Anmeldenummer: **81810283.2**

(22) Anmeldetag: **13.07.81**

(51) Int. Cl.³: **A 01 N 47/36,** C 07 D 251/16, C 07 D 251/44, C 07 D 251/46

(54) **N-(2-substituierte Phenylsulfonyl)-N'-triazinylharnstoffe.**

(30) Priorität: **17.07.80 CH 5481/80**
**05.11.80 CH 8216/80**
**17.06.81 CH 3991/81**

(43) Veröffentlichungstag der Anmeldung:
**27.01.82 Patentblatt 82/4**

(45) Bekanntmachung des Hinweises auf die Patenterteilung:
**31.08.83 Patentblatt 83/35**

(84) Benannte Vertragsstaaten:
**AT BE DE FR GB IT NL SE**

(56) Entgegenhaltungen:
**EP-A-0 001 514**
**EP-A-0 001 515**
**EP-A-0 023 422**

(73) Patentinhaber: **CIBA-GEIGY AG, Patentabteilung Postfach, CH-4002 Basel (CH)**

(72) Erfinder: **Meyer, Willy, Talstrasse 49, CH-4125 Riehen (CH)**
Erfinder: **Föry, Werner, Dr., Benkenstrasse 65, CH-4054 Basel (CH)**

EP 0 044 809 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

# N-(2-substituierte Phenylsulfonyl)-N′-triazinylharnstoffe

Die vorliegende Erfindung betrifft neue, herbizid wirksame und pflanzenwuchsregulierende N-(2-substituierte Phenylsulfonyl)-N′-triazinylharnstoffe, Verfahren zu ihrer Herstellung, sie als Wirkstoffe enthaltende Mittel sowie deren Verwendung zum Bekämpfen von Unkräutern, vor allem selektiv in Nutzpflanzenkulturen oder zum Regulieren und Hemmen des Pflanzenwachstums.

Die erfindungsgemässen N-(2-substituierten Phenylsulfonyl)-N′-triazinyl-harnstoffe entsprechen der allgemeinen Formel I

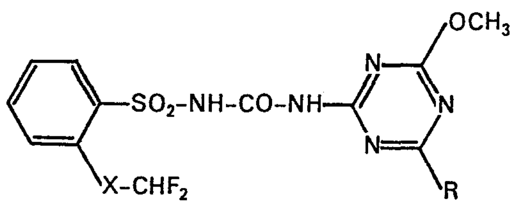 (I),

worin X Sauerstoff und R Methyl oder Methoxy bedeuten, und ausserdem X für Schwefel stehen kann, wenn R Methyl bedeutet, sowie den Salzen dieser Verbindungen.

Harnstoffverbindungen, Triazinverbindungen und Pyrimidinverbindungen mit herbizider Wirkung sind allgemein bekannt. Kürzlich wurden Arylsulfamoyl-heterocyclyl-aminocarbamoylverbindungen mit herbizider und pflanzwuchsregulierender Wirkung, beispielsweise in den europäischen Patentpublikationen Nr. 1514, 1515, dem US Patent No. 4 127 405, in der DT-OS 2 715 786 oder in der FR-PS 1 468 747 beschrieben. In der nicht veröffentlichten europäischen Patentpublikation Nr. 23 422 wird eine Gruppe von N-(2-substituierten Phenylsulfonyl)-N′-triazinyl-harnstoffen beschrieben, die auch die erfindungsgemässen Verbindungen einschliesst. Die erfindungsgemässen Verbindungen und ihre besonderen Eigenschaften sind darin jedoch nicht erwähnt.

Die Erfindung umfasst ebenfalls die Salze, die die Verbindungen der Formel I mit Aminen, Alkali- und Erdalkalimetallionen oder quaternären Ammoniumionen bilden können.

Unter Alkali- und Erdalkalimetallhydroxyden als Salzbildner sind die Hydroxyde von Lithium, Natrium, Kalium, Magnesium oder Calcium hervorzuheben, insbesondere aber die von Natrium oder Kalium.

Beispiele für zur Salzbildung geeignete Amine sind primäre, sekundäre und tertiäre aliphatische und aromatische Amine wie Methylamin, Äthylamin, Propylamin, i-Propylamin, die vier isomeren Butylamine, Dimethylamin, Diäthylamin, Diäthanolamin, Dipropylamin, Diisopropylamin, Di-n-butylamin, Pyrrolidin, Piperidin, Morpholin, Trimethylamin, Triäthylamin, Tripropylamin, Chinuclidin, Pyridin, Chinolin und i-Chinolin, insbesondere aber Äthyl-, Propyl-, Diäthyl- oder Triäthylamin, vor allem aber iso-Propylamin und Diäthanolamin.

Beispiele für quaternäre Ammoniumionen sind im allgemeinen die Kationen von Halogenammoniumsalzen, z.B. das Tetramethylammoniumkation, das Trimethylbenzylammoniumkation, das Triäthylbenzylammoniumkation, das Tetraäthylammoniumkation, das Trimethyläthylammoniumkation, aber auch das Ammoniumkation.

Die Herstellung der Verbindungen der Formel I erfolgt nach an sich bekannten Methoden.

Nach einem ersten Verfahren werden die Verbindungen der Formel I erhalten, indem man ein Phenylsulfonamid der Formel II

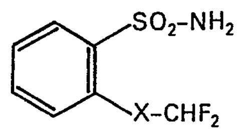 (II),

worin X die unter Formel I gegebene Bedeutung hat, in Gegenwart einer Base mit einem N-Triazinyl-carbamat der Formel III

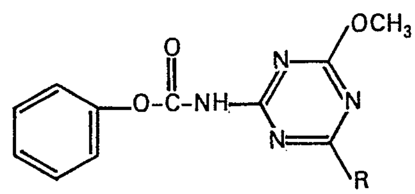 (III),

worin R die unter Formel I gegebene Bedeutung hat, umsetzt.

Nach einem zweiten Verfahren gelangt man zu Verbindungen der Formel I, indem man ein Phenylsulfonylisocyanat der Formel IV

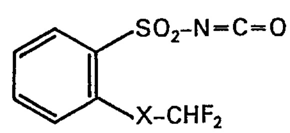 (IV),

worin X die unter der Formel I gegebene Bedeutung hat, gegebenenfalls in Gegenwart einer Base, mit einem Amin der Formel V

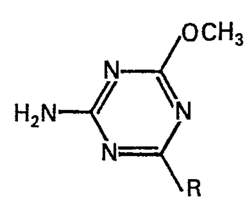 (V),

worin R die unter Formel I gegebene Bedeutung hat, umsetzt.

Nach einem weiteren Verfahren werden die Verbindungen der Formel I hergestellt, indem man ein Sulfonamid der oben angegebenen Formel II gegebenenfalls in Gegenwart einer Base mit einem Isocyanat der Formel VI

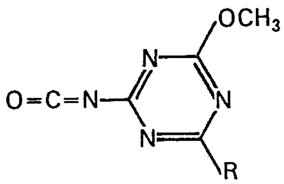

(VI),

worin R die unter Formel I gegebene Bedeutung hat, umsetzt.

Schliesslich kann man die Verbindungen der Formel I auch erhalten, indem man ein N-Phenylsulfonylcarbamat der Formel VII

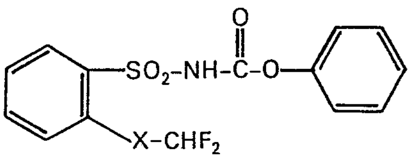

(VII),

worin X die unter Formel I gegebene Bedeutung hat, mit einem Amin der oben angegebenen Formel V umsetzt.

Die erhaltenen Harnstoffe der Formel I können gewünschtenfalls mittels Aminen, Alkalimetall- oder Erdalkalimetallhydroxiden oder quaternären Ammoniumbasen in basische Additionssalze übergeführt werden. Dieses geschieht beispielsweise durch Umsetzen mit der äquimolaren Menge Base und Verdampfen des Lösungsmittels.

Die als Zwischenprodukte verwendeten Sulfonamide der Formel II werden aus den entsprechenden Anilinen durch Diazotierung und Austausch der Diazogruppe mit Schwefeldioxid in Gegenwart eines Katalysators wie Kupfer-I-chlorid in Salzsäure und Umsetzen des entstandenen Phenylsulfonylchlorids mit Ammoniumhydroxid-Lösung erhalten.

Die Phenylsulfonylisocyanate der Formel IV können durch Umsetzungen der Sulfonamide der Formel II mit Phosgen in Anwesenheit von Alkylisocyanat in einem chlorierten Kohlenwasserstoff als Lösungsmittel, bei Rückflusstemperatur erhalten werden. Ähnliche Darstellungen sind in «Newer Methods of Preparative Organic Chemistry» Band VI, 223-241, Academic Press New York und London, beschrieben.

Die N-Phenylsulfonylcarbamate der Formel VII sind neu und werden durch Umsetzung der Sulfonamide der Formel II mit Diphenylcarbamat in Gegenwart einer Base erhalten. Ähnliche Verfahren sind in der japanischen Patentschrift 61 169 beschrieben.

Die Ausgansmaterialien der Formeln III, V und VI sind bekannt oder können nach bekannten Methoden hergestellt werden.

Amine der Formel V sind bekannt und zum Teil im Handel erhältlich oder sie können nach bekannten Methoden hergestellt werden, siehe «The Chemistry of Heterocyclic Compounds» Band XIV, Interscience Publishers New York, London.

Diese Umsetzungen werden vorteilhafterweise in aprotischen, inerten, organischen Lösungsmitteln vorgenommen wie Methylenchlorid, Tetrahydrofuran, Acetonitril, Dioxan, Toluol.

Die Reaktionstemperaturen liegen vorzugsweise zwischen −20° und +120°C. Die Umsetzungen verlaufen im allgemeinen leicht exotherm und können bei Raumtemperatur durchgeführt werden. Zwecks Abkürzung der Reaktionszeit oder auch zum Einleiten der Umsetzung wird zweckdienlich für kurze Zeit bis zum Siedepunkt des Reaktionsgemisches aufgewärmt. Die Reaktionszeiten können ebenfalls durch Zugabe einiger Tropfen Base oder Isocyanat als Reaktionskatalysator beschleunigt werden.

Die Endprodukte können durch Einengen und/oder Verdampfen des Lösungsmittels isoliert und durch Umkristallisieren oder Zerreiben des festen Rückstandes in Lösungsmitteln, in denen sie sich nicht gut lösen wie Äther, aromatischen Kohlenwasserstoffen oder chlorierten Kohlenwasserstoffen, gereinigt werden.

Die Wirkstoffe der Formel I sind stabile Verbindungen. Ihre Handhabung bedarf keiner vorsorglicher Massnahmen.

Die Verbindungen der Formel I haben starke pflanzenwuchsregulierende, insbesondere pflanzenwuchshemmende, Eigenschaften. Es werden sowohl Monokotyledonen als auch Dikotyledonen in ihrem Wachstum beeinträchtigt.

So können z.B. die in der Landwirtschaft in tropischen Gegenden häufig als «cover crops» (Bodenbedecker) angepflanzten Leguminosen durch die Verbindungen der Formel I in ihrem Wachstum selektiv gehemmt werden, so dass zwar die Bodenerosion zwischen den Kulturpflanzen verhindert wird, die «cover crops» jedoch nicht zur Konkurrenz für die Kultur werden können.

Weiter eignen sich die Verbindungen der Formel I, um das Keimen von eingelagerten Kartoffeln zu verhindern. Bei Kartoffeln entwickeln sich bei der Einlagerung über den Winter häufig Keime, die Schrumpfen, Gewichtsverlust und Faulen zur Folge haben.

Bei grösseren Aufwandmengen werden alle getesteten Pflanzen in ihrer Entwicklung so geschädigt, dass sie absterben.

Bei geringeren Aufwandmengen zeichnen sich die Verbindungen der Formel I durch gute selektiv-wuchshemmende und selektiv-herbizide Eigenschaften aus, die sie ausgezeichnet zum Einsatz in Kulturen von Nutzpflanzen, insbesondere in Getreide und Reis, befähigen. Es werden dabei teilweise auch Unkräuter geschädigt, welchen bisher nur mit Totalherbiziden beizukommen war.

Die Wirkungsart dieser Wirkstoffe ist unüblich, sie sind oft translozierbar, d.h. sie werden von der Pflanze aufgenommen und an andere Stellen transportiert, wo sie dann zur Wirkung kommen. Das Unübliche daran ist, dass sie nicht nur den Weg der Nährstoffe durch die Gefässbündel im Holzteil, von den Wurzeln in die Blätter nehmen, sondern auch durch die Siebröhren im Bastteil von den Blättern zurück in die Wurzel transloziert werden können. So gelingt es beispielsweise, durch Oberflächenbehandlung perenierende Unkräuter bis in die Wurzeln zu schädigen. Die neuen Verbindungen der Formel I wirken bereits

bei – im Vergleich zu anderen Herbiziden und Wuchsregulatoren – sehr geringen Aufwandmengen.

Die Erfindung betrifft auch herbizide und pflanzenwachstumsregulierende Mittel, welche einen neuen Wirkstoff der Formel I erhalten, sowie Verfahren zur pre- und post-emergenten Unkrautbekämpfung und zur Hemmung des Pflanzenwuchses von monokotylen und dikotylen Pflanzen, insbesondere Gräsern, tropischen Bodenbedeckern und Tabakgeiztrieben.

Die Verbindungen der Formel I werden in unveränderter Form oder vorzugsweise als Mittel zusammen mit den in der Formulierungstechnik üblichen Hilfsmitteln eingesetzt und werden daher z.B. zu Emulsionskonzentraten, direkt versprühbaren oder verdünnbaren Lösungen, verdünnten Emulsionen, Spritzpulvern, löslichen Pulvern, Stäubemitteln, Granulaten, auch Verkapselungen in z.B. polymeren Stoffen in bekannter Weise verarbeitet. Die Anwendungsverfahren wie Versprühen, Vernebeln, Verstäuben, Verstreuen oder Giessen werden gleich wie die Art der Mittel den angestrebten Zielen und den gegebenen Verhältnissen entsprechend gewählt.

Die Formulierungen, d.h. die den Wirkstoff der Formel I und gegebenenfalls einen festen oder flüssigen Zusatzstoff enthaltenden Mittel, Zubereitungen oder Zusammensetzungen, werden in bekannter Weise hergestellt, z.B. durch inniges Vermischen und/oder Vermahlen der Wirkstoffe mit Streckmitteln, wie z.B. mit Lösungsmitteln, festen Trägerstoffen, und gegebenenfalls oberflächenaktiven Verbindungen (Tensiden).

Als Lösungsmittel können in Frage kommen: Aromatische Kohlenwasserstoffe, bevorzugt die Fraktionen $C_8$ bis $C_{12}$, wie z.B. Xylolgemische oder substituierte Naphthaline, Phthalsäureester wie Dibutyl- oder Dioctylphthalat, aliphatische Kohlenwasserstoffe wie Cyclohexan oder Paraffine, Alkohole und Glykole sowie deren Äther und Ester wie Äthanol, Äthylenglykol, Äthylenglykolmonomethyl- oder Athyläther, Ketone wie Cyclohexanon, stark polare Lösungsmittel wie N-Methyl-2-pyrrolidon, Dimethylsulfoxid oder Dimethylformamid sowie gegebenenfalls epoxidierte Pflanzenöle wie epoxidiertes Kokusnussöl oder Sojaöl oder Wasser.

Als feste Trägerstoffe, z.B. für Stäubemittel und dispergierbare Pulver, werden in der Regel natürliche Gesteinsmehle verwendet wie Calcit, Talkum, Kaolin, Montmorillonit oder Attapulgit. Zur Verbesserung der physikalischen Eigenschaften können auch hochdisperse Kieselsäure oder hochdisperse saugfähige Polymerisate zugesetzt werden. Als gekörnte, adsorptive Granulatträger kommen poröse Typen wie z.B. Bimsstein, Ziegelbruch, Sepiolit oder Bentonit, als nicht sorptive Trägermaterialien z.B. Calcit oder Sand in Frage. Darüberhinaus kann eine Vielzahl von vorgranulierten Materialien anorganischer oder organischer Natur wie insbesondere Dolomit oder zerkleinerte Pflanzenrückstände verwendet werden.

Als oberflächenaktive Verbindungen kommen je nach der Art des zu formulierenden Wirkstoffes der Formel I nichtionogene, kation- und/oder anionaktive Tenside mit guten Emuglier-, Dispergier- und Netzeigenschaften in Betracht. Unter Tensiden sind auch Tensidgemische zu verstehen.

Geeignete anionische Tenside können sowohl sog. wasserlösliche Seifen als auch wasserlösliche synthetische oberflächenaktive Verbindungen sein.

Als Seifen seien die Alkali-, Erdalkali- oder gegebenenfalls substituierte Ammoniumsalze von höheren Fettsäuren ($C_{10}$-$C_{22}$) wie z.B. die Na- oder K-Salze der Öl- oder Stearinsäure oder von natürlichen Fettsäuregemischen, die z.B. aus Kokosnuss- oder Talgöl gewonnen werden können, genannt. Ferner sind auch die Fettsäure-methyltaurinsalze zu erwähnen.

Häufiger werden jedoch sogenannte synthetische Tenside verwendet, insbesondere Fettsulfonate, Fettsulfate, sulfonierte Benzimidazolderivate oder Alkylarylsulfonate.

Die Fettsulfonate oder -sulfate liegen in der Regel als Alkali-, Erdalkali oder gegebenenfalls substituierte Ammoniumsalze vor und weisen einen Alkylrest mit 8 bis 22 C-Atomen auf, wobei Alkyl auch den Alkylteil von Acylresten einschliesst, z.B. das Na- oder Ca-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkohol-Äthylenoxid-Addukten. Die sulfonierten Benzimidazolderivate enthalten vorzugsweise 2 Sulfonsäuregruppen und einen Fettsäurerest mit 8–22 C-Atomen. Alkylarylsulfonate sind z.B. die Na-, Ca- oder Triäthanolaminsalze der Dodecylbenzolsulfonsäure, der Dibutylnaphthalinsulfonsäure, oder eines Naphthalinsulfonsäure-Formaldehydkondensationsproduktes.

Ferner kommen auch entsprechende Phosphate wie z.B. Salze des Phosphorsäureesters eines p-Nonylphenol-(4-14)-Äthylenoxid-Adduktes in Frage.

Als nichtionische Tenside kommen in erster Linie Polyglykolätherderivate von aliphatischen oder cycloaliphatischen Alkoholen, gesättigten oder ungesättigten Fettsäuren und Alkylphenolen in Frage, die 3 bis 30 Glykoläthergruppen und 8 bis 20 Kohlenstoffatome im (aliphatischen) Kohlenwasserstoffrest und 6 bis 18 Kohlenstoffatome im Alkylrest der Alkylphenole enthalten können.

Weitere geeignete nichtionische Tenside sind die wasserlöslichen, 20 bis 250 Äthylenglykoläthergruppen und 10 bis 100 Propylenglykoläthergruppen enthaltenden Polyäthylenoxidaddukte an Polypropylenglykol, Äthylendiaminopolypropylenglykol und Alkylpolypropylenglykol mit 1 bis 10 Kohlenstoffatomen in der Alkylkette. Die genannten Verbindungen enthalten üblicherweise pro Propylenglykol-Einheit 1 bis 5 Äthylenglykoleinheiten.

Als Beispiele nichtionischer Tenside seien Nonylphenolpolyäthoxyäthanole, Ricinusölpolyglykoläther, Polypropylen-Polyäthylenoxidaddukte, Tributylphenoxypolyäthoxyäthanol, Polyäthylenglykol und Octylphenoxypolyäthoxyäthanol erwähnt.

Ferner kommen auch Fettsäureester von Polyoxyäthylensorbitan wie das Polyoxyäthylensorbitan-trioleat in Betracht.

Bei den kationischen Tensiden handelt es sich vor allem um quartäre Ammoniumsalze, welche als N-Substituenten mindestens einen Alkylrest mit 8 bis 22 C-Atomen enthalten und als weitere Substituenten niedrige, gegebenenfalls halogenierte Alkyl-, Benzyl- oder niedrige Hydroxyalkylreste aufweisen. Die Salze liegen vorzugsweise als Halogenide, Methylsulfate oder Äthylsulfate vor, z.B. das Stearyltrimethylammoniumchlorid oder das Benzyldi(2-chloräthyl)äthylammoniumbromid.

Die in der Formulierungstechnik gebräuchlichen Tenside sind u.a. in folgenden Publikationen beschrieben:

«Mc Cutcheon's Detergents and Emulsifiers Annual» MC Publishing Corp., Ridgewood, New Jersey, 1979.
Sisley and Wood, «Encyclopedia of Surface Active Agents», Chemical Publishing Co., Inc. New York, 1964.

Die pestiziden Zubereitungen enthalten in der Regel 0,1 bis 95%, insbesondere 1 bis 80%, Wirkstoff der Formel I, 1 bis 99% eines festen oder flüssigen Zusatzstoffes und 0 bis 25%, insbesondere 0,1 bis 25% eines Tensides.

Insbesondere setzen sich bevorzugte Formulierungen folgendermassen zusammen: (% = Gewichtsprozent)

Lösungen
Aktiver Wirkstoff: 5 bis 95%, vorzugsweise 10 bis 80%
Lösungsmittel: 95 bis 5%, vorzugsweise 90 bis 0%
oberflächenaktives Mittel: 1 bis 30%, vorzugsweise 2 bis 20%.

Emulgierbare Konzentrate:
Aktiver Wirkstoff: 10 bis 50%, bevorzugt 10 bis 40%
oberflächenaktives Mittel: 5 bis 30%, vorzugsweise 10 bis 20%
flüssiges Trägermittel: 20 bis 95%, vorzugsweise 40 bis 80%.

Stäube
Aktiver Wirkstoff: 0,5 bis 10%, vorzugsweise 2 bis 8%
festes Trägermittel: 99,5 bis 90%, vorzugsweise 98 bis 92%.

Suspension-Konzentrate
Aktiver Wirkstoff: 5 bis 75%, vorzugsweise 10 bis 50%
Wasser: 94 bis 25%, vorzugsweise 90 bis 30%
oberflächenaktives Mittel: 1 bis 40%, vorzugsweise 2 bis 30%.

Benetzbare Pulver
Aktiver Wirkstoff: 5 bis 90%, vorzugsweise 10 bis 80% und insbesondere 20 bis 60%
oberflächenaktives Mittel: 0,5 bis 20%, vorzugsweise 1 bis 15%
festes Trägermittel: 5 bis 90%, vorzugsweise 30 bis 70%.

Granulate
Aktiver Wirkstoff: 0,5 bis 30%, vorzugsweise 3 bis 15%
festes Trägermittel: 99,5 bis 70%, vorzugsweise 97 bis 85%.

Während als Handelsware eher konzentrierte Mittel bevorzugt werden, verwendet der Endverbraucher in der Regel verdünnte Mittel. Die Anwendungsformen können bis hinab zu 0,001% an Wirkstoff verdünnt werden. Die Aufwandmengen betragen in der Regel 0,01 bis 10 kg AS/ha, vorzugsweise 0,025 bis 5 kg AS/ha.

Die Mittel können auch weitere Zusätze wie Stabilisatoren, Entschäumer, Viskositätsregulatoren, Bindemittel, Haftmittel sowie Dünger oder andere Wirkstoffe zur Erzielung spezieller Effekte enthalten.

In den folgenden Beispielen sind die Temperaturen in Celsiusgraden °C, die Drücke in Millibar mb angegeben.

Herstellungsbeispiele:
Beispiel 1

a) 2-Difluormethoxyphenyl-sulfonylchlorid.

Zu einer Lösung von 6,4 g Kupfersulfat in 276 ml 36%iger Salzsäure und 72 ml Wasser lässt man unter Kühlung bei 0° 101,2 g einer 40%igen Natriumhydrogensulfit-Lösung zutropfen. Zu der erhaltenen Lösung lässt man gleichzeitig 101,2 g einer 40%igen Natriumhydrogensulfit-Lösung und eine auf −10° gekühlte Diazoniumsalz-Lösung, die man durch Zutropfenlassen unter Kühlung von 28,8 g Natriumnitrit in 44 ml Wasser zu einer Lösung von 39 ml Wasser, 84 ml 36%iger Salzsäure und 63,7 g 2-Difluormethoxy-anilin erhält, zutropfen. Dabei erwärmt sich das Reaktionsgemisch unter gleichzeitiger Gasentwicklung auf 30°. Nach Rühren des Reaktionsgemisches für 18 Stunden scheidet sich ein braunes Öl ab. Nach Abtrennen der organischen Phase extrahiert man die wässrige Phase mit Methylenchlorid. Durch Vereinigen und Eindampfen der organischen Phasen erhält man als Rohprodukt 80,3 g 2-Difluormethoxyphenyl-sulfonylchlorid als braunes Öl.

b) 2-Difluormethoxyphenyl-sulfonamid.

Einer Mischung aus 250 ml Methylenchlorid, 250 ml Wasser und 250 ml 30%iger Ammoniak-Lösung fügt man langsam eine Lösung von 80,3 g rohem 2-Difluormethoxyphenyl-sulfonyl-chlorid in 50 ml Methylenchlorid zu. Nach Abklingen der exothermen Reaktion wird die Reaktionsmischung für 2 Stunden unter Rückfluss gekocht. Nach Waschen der organischen Phase mit Wasser, Trocknen über Natriumsulfat, Eindampfen und Umkristallisieren aus Methanol erhält man

40,5 g 2-Difluormethoxyphenyl-sulfonamid vom Schmelzpunkt 128–130°.

c) N-(2-Difluormethoxyphenyl-sulfonyl)-phenyl-carbamat.

Zu einer auf 0°-10° gekühlten Suspension von 0,84 g 57,5%iger Natriumhydrid-Dispersion in 5 ml absolutem Dimethylformamid lässt man eine Lösung von 4,5 g 2-Difluormethoxy-phenylsulfonamid in 5 ml absolutem Dimethylformamid zutropfen. Zur Vervollständigung der Wasserstoffentwicklung erwärmt man die Reaktionsmischung unter Rühren auf 25°. Nach beendeter Wasserstoffentwicklung und Abkühlen der Mischung auf −30° wird eine Lösung von 4,7 g Diphenylcarbonat in 5 ml absolutem Dimethylformamid zugesetzt und danach die Reaktionsmischung unter Erwärmenlassen bis auf 25° für 18 Stunden gerührt. Nach Eingiessen der Reaktionslösung in ein Gemisch aus 11 ml 2 N Salzsäure und 100 g Eis nimmt man das ausgefallene Produkt mit Äthylacetat auf. Durch Trocknen und Eindampfen der Äthylacetat-Phase und Umkristallisieren aus Methanol erhält man 5,6 g N-(2-Difluormethoxyphenyl-sulfonyl)-phenyl-carbamat als farblose Kristalle vom Schmelzpunkt 115–117°.

d) N-(2-Difluormethoxyphenyl-sulfonyl)-N′-(4-methoxy-6-methyl-1,3,5-triazin-2-yl)-harnstoff.

Eine für 5 Stunden unter Rückfluss gekochte Lösung von 5,4 g N-(2-Difluormethoxyphenyl-sulfonyl)-phenyl-carbamat und 2,2 g 2-Amino-4-methoxy-6-methyl-1,3,5-triazin in 60 ml absolutem Dioxan wird in 600 ml Eiswasser gegossen. Das ausgefallene Produkt wird mit Äthylacetat aufgenommen. Das nach Waschen mit Wasser, Trocknen und Eindampfen der Äthylacetat-Phase erhaltene Öl wird erneut in Äthylacetat gelöst und fünfmal mit gesättigter Natriumhydrogencarbonat-Lösung gewaschen. Die vereinigten wässrigen Phasen werden mit verdünnter Salzsäure angesäuert und mit Äthylacetat extrahiert. Nach Trocknen und Eindampfen der Äthylacetat-Phase und Umkristallisieren aus Methanol erhält man 5,0 g N-(2-Difluormethoxyphenyl-sulfonyl)-N′-(4-methoxy-6-methyl-1,3,5-triazin-2-yl)-harnstoff vom Schmelzpunkt 132–134° (Verbindung 1).

Beispiel 2

N-(2-Difluormethylthiophenyl-sulfonyl)-N′-(4-methoxy-6-methyl-1,3,5-triazin-2-yl)-harnstoff.

Analog den Beispielen 1a–d erhält man aus 2-Difluormethylthioanilin über das Sulfonylchlorid das 2-Difluormethylthiophenylsulfonamid, Smp. 141–142°C. Dieses Sulfonamid wird in das entsprechende N-(2-Difluormethylthiophenyl-sulfonyl)-phenyl-carbamat und durch Umsetzen mit 2-Amino-4-methoxy-6-methyl-1,3,5-triazin in N-(2-Difluormethylthiophenyl-sulfonyl)-N′-(4-methoxy-6-methyl-1,3,5-triazin-2-yl)-harnstoff überführt, Smp. 169–172°C (Verbindung 2).

Beispiel 3

a) N-(2-Difluormethoxyphenyl-sulfonyl)-N-(4,6-dichlor-1,3,5-triazin-2-yl)-harnstoff.

Einer Lösung von 3,9 g 4,6-Dichlor-2-isocyanato-1,3,5-triazin in 50 ml absolutem Dioxan werden 4,5 g 2-Difluormethoxyphenyl-sulfonamid zugesetzt. Nach 5 Stunden Kochen unter Rückfluss wird die entstandene klare Lösung in 500 ml Eiswasser gegossen. Das ausgefallene Produkt wird mit Äthylacetat extrahiert. Durch Trocknen und Einengen der organischen Phase erhält man als Kristallisat vom Schmelzpunkt 105–107° 8,5 g eines 1:1-Adduktes von Dioxan und N-(2-Difluormethoxyphenyl-sulfonyl)-N-(4,6-dichlor-1,3,5-triazin-2-yl)-harnstoff.

b) N-(2-Difluormethoxyphenyl-sulfonyl)-N′-(4,6-dimethoxy-1,3,5-triazin-2-yl)-harnstoff.

Zur Lösung von 5,5 g des nach Beispiel 3a erhaltenen N-(2-difluormethoxyphenyl-sulfonyl)-N′-(4,6-dichlor-1,3,5-triazin-2-yl)-harnstoff-Dioxan-Adduktes in 10 ml absolutem Methanol lässt man innerhalb von 15 Minuten eine Lösung von 1,5 g Natriummethylat in 10 ml absolutem Methanol zutropfen. Nach 2 Stunden Kochen unter Rückfluss wird die Reaktionsmischung eingedampft und der Rückstand in 200 ml Eiswasser gelöst. Beim Ansäuern der Lösung mit verdünnter Salzsäure fallen 3,2 g N-(2-Difluormethoxyphenyl-sulfonyl)-N′-(4,6-dimethoxy-1,3,5-triazin-2-yl)-harnstoff als farblose Kristalle vom Schmelzpunkt 135–137° an (Verbindung 3).

Formulierungsbeispiele:

Beispiel 4

(% = Gewichtsprozent)

| a) Spritzpulver | a) | b) |
|---|---|---|
| Wirkstoff | 20% | 60% |
| Na-Ligninsulfonat | 5% | 5% |
| Na-Laurylsulfat | 3% | - |
| Na-Diisobutylnaphthalinsulfonat | - | 6% |
| Octylphenolpolyäthylenglykoläther (7–8 Mol AeO) | - | 2% |
| Hochdisperse Kieselsäure | 5% | 27% |
| Kaolin | 67% | - |

Der Wirkstoff wird mit den Zusatzstoffen gut vermischt und in einer geeigneten Mühle gut vermahlen. Man erhält Spritzpulver, die sich mit Wasser zu Suspensionen jeder gewünschten Konzentration verdünnen lassen.

| b) Emulsions-Konzentrat | |
|---|---|
| Wirkstoff | 10% |
| Octylphenolpolyäthylenglykoläther (4–5 Mol AeO) | 3% |
| Ca-Dodecylbenzolsulfonat | 3% |
| Ricinusölpolyglykoläther (36 Mol AeO) | 4% |
| Cyclohexanon | 30% |
| Xylolgemisch | 50% |

Aus diesem Konzentrat können durch Verdünnen mit Wasser Emulsionen jeder gewünschten Konzentration hergestellt werden.

| c) Stäubemittel | a) | b) |
|---|---|---|
| Wirkstoff | 5% | 8% |
| Talkum | 95% | - |
| Kaolin | - | 92% |

Man erhält anwendungsfertige Stäubemittel, indem der Wirkstoff mit dem Träger vermischt und auf einer geeigneten Mühle vermahlen wird.

d) Extruder Granulat

| | |
|---|---|
| Wirkstoff | 10% |
| Na-Ligninsulfonat | 2% |
| Carboxymethylcellulose | 1% |
| Kaolin | 87% |

Der Wirkstoff wird mit den Zusatzstoffen vermischt, vermahlen und mit Wasser angefeuchtet. Dieses Gemisch wird extrudiert und anschliessend im Luftstrom getrocknet.

e) Umhüllungs-Granulat

| | |
|---|---|
| Wirkstoff | 3% |
| Polyäthylenglykol | 3% |
| Kaolin | 94% |

Der fein gemahlene Wirkstoff wird in einem Mischer auf das mit Polyäthylenglykol angefeuchtete Kaolin gleichmässig aufgetragen. Auf diese Weise erhält man staubfreie Umhüllungs-Granulate.

f) Suspensions-Konzentrat

| | |
|---|---|
| Wirkstoff | 40% |
| Äthylenglykol | 10% |
| Nonylphenolpolyäthylenglykoläther (15 Mol AeO) | 6% |
| Na-Ligninsulfonat | 10% |
| Carboxymethylcellulose | 1% |
| 37%ige wässrige Formaldehyd-Lösung | 0,2% |
| Silikonöl in Form einer 75%igen wässrigen Emulsion | 0,8% |
| Wasser | 32,5% |

Der fein gemahlene Wirkstoff wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

Biologische Beispiele

Beispiel 5

Nachweis der Herbizidwirkung vor dem Auflaufen der Pflanzen

Im Gewächshaus werden Pflanzensamen in Blumentöpfe von 12–15 cm Durchmesser gesät. Unmittelbar danach wird die Erdoberfläche mit einer wässrigen Dispersion oder Lösung der Wirkstoffe behandelt. Es werden verschiedene Konzentrationen mit Wirkstoffmengen pro Hektar angewendet. Die Töpfe werden dann im Gewächshaus bei einer Temperatur von 22–25°C und 50–70% relativer Luftfeuchtigkeit gehalten. Nach 3 Wochen wird der Versuch ausgewertet und die Wirkung nach folgendem Massstab beurteilt:

1: Pflanzen nicht gekeimt oder total abgestorben,
2–3: sehr starke Wirkung,
4–6: mittlere Wirkung
7–8: geringe Wirkung,
9: keine Wirkung (wie unbehandelte Kontrolle).

**Versuchsergebnisse** (preemergent):

| Wirkung<br>Aufwandmenge<br>kg AS/ha | Verb.<br>Nr. 1 | | | | Verb.<br>Nr. 2 | | | | Verb.<br>Nr. 3 | | | |
|---|---|---|---|---|---|---|---|---|---|---|---|---|
| Testpflanze | 0,12 | 0,06 | 0,03 | 0,015 | 0,5 | 0,25 | 0,12 | 0,06 | 0,25 | 0,12 | 0,06 | 0,03 |
| Gerste | 5 | 6 | 6 | 7 | 7 | 8 | 8 | 8 | 7 | 8 | 9 | 9 |
| Weizen | 6 | 7 | 7 | 8 | 9 | 9 | 9 | 9 | 9 | 9 | 9 | 9 |
| Lolium perenne | 1 | 1 | 1 | 1 | 2 | 2 | 2 | 3 | 2 | 3 | 4 | 6 |
| Alopecurus myos | 1 | 1 | 1 | 2 | 2 | 2 | 2 | 2 | 2 | 3 | 4 | 4 |
| Rottboellia ex. | 3 | 3 | 3 | 4 | 1 | 1 | 1 | 2 | 5 | 6 | 7 | 8 |
| Abutilon | 1 | 2 | 2 | 2 | 1 | 1 | 1 | 1 | 2 | 2 | 2 | 3 |
| Sida spinosa | 2 | 2 | 2 | 2 | 1 | 1 | 1 | 1 | 2 | 2 | 2 | 2 |
| Amaranthus ret. | 1 | 1 | 1 | 1 | 1 | 2 | 2 | 2 | 1 | 1 | 1 | 2 |
| Chenopodium sp. | 2 | 2 | 2 | 2 | 1 | 1 | 2 | 2 | 2 | 2 | 2 | 2 |
| Solanum nigrum | 2 | 2 | 2 | 2 | 1 | 1 | 2 | 2 | 1 | 2 | 3 | 3 |
| Ipomoea | 2 | 2 | 2 | 2 | 1 | 1 | 1 | 2 | 2 | 2 | 3 | 5 |
| Sinapis | 1 | 1 | 1 | 1 | 2 | 2 | 2 | 2 | 2 | 2 | 2 | 2 |
| Stellaria | 1 | 1 | 1 | 1 | 2 | 2 | 2 | 2 | 2 | 2 | 2 | 2 |
| Chrysanthe. leuc. | 1 | 1 | 1 | 1 | 2 | 2 | 2 | 2 | 2 | 2 | 2 | 2 |
| Galium aparine | 1 | 1 | 3 | 4 | 2 | 2 | 3 | 4 | 2 | 2 | 3 | 3 |
| Viola tricolor | 1 | 1 | 1 | 1 | 2 | 2 | 2 | 2 | 2 | 2 | 2 | 3 |
| Veronica Sp. | 1 | 1 | 1 | 1 | 1 | 1 | 1 | 1 | 2 | 2 | 2 | 3 |

Beispiel 6

Nachweis der Herbizidwirkung nach dem Auflaufen der Pflanzen (Kontaktwirkung).

Eine grössere Anzahl Unkräuter und Kulturpflanzen, sowohl monocotyle wie dicotyle, wurden nach dem Auflaufen, im 4- bis 6-Blattstadium mit einer wässrigen Wirkstoffdispersion in verschiedenen Dosierungen gespritzt und dann bei

24° bis 26°C und 45–60% relativer Luftfeuchtigkeit gehalten. 15 Tage nach der Behandlung wird der Versuch ausgewertet und die Wirkung nach dem gleichen Massstab wie im Beispiel 5 bewertet.

**Versuchsergebnisse** (postemergent):

| Wirkung<br>Aufwandmenge<br>kg AS/ha<br>Testpflanze | Verb.<br>Nr. 1<br><br>0,12 | <br><br><br>0,06 | <br><br><br>0,03 | <br><br><br>0,015 | Verb.<br>Nr. 2<br><br>0,5 | <br><br><br>0,25 | <br><br><br>0,12 | <br><br><br>0,06 | Verb.<br>Nr. 3<br><br>0,25 | <br><br><br>0,12 | <br><br><br>0,06 | <br><br><br>0,3 |
|---|---|---|---|---|---|---|---|---|---|---|---|---|
| Gerste | 6 | 7 | 9 | 9 | 7 | 9 | 9 | 9 | 8 | 9 | 9 | 9 |
| Weizen | 7 | 9 | 9 | 9 | 7 | 9 | 9 | 9 | 9 | 9 | 9 | 9 |
| Lolium perenne | 2 | 2 | 2 | 2 | 7 | 9 | 9 | 9 | 3 | 3 | 3 | 3 |
| Alopecurus myos | 3 | 3 | 3 | 3 | 4 | 4 | 6 | 6 | 3 | 4 | 4 | 5 |
| Rottboellia ex. | 6 | 6 | 6 | 6 | 6 | 8 | 9 | 9 | 4 | 6 | 6 | 7 |
| Abutilon | 1 | 1 | 3 | 4 | 3 | 4 | 4 | 4 | 2 | 2 | 3 | 3 |
| Sida spinosa | 8 | 8 | 8 | 9 | 7 | 7 | 7 | 9 | 7 | 8 | 8 | 8 |
| Amaranthus ret. | 1 | 1 | 2 | 2 | 2 | 2 | 2 | 3 | 2 | 3 | 3 | 3 |
| Chenopodium sp. | 1 | 1 | 1 | 1 | 1 | 1 | 2 | 2 | 2 | 2 | 4 | 4 |
| Solanum nigrum | 2 | 2 | 3 | 3 | 4 | 4 | 4 | 4 | 5 | 6 | 6 | 7 |
| Ipomoea | 3 | 3 | 3 | 4 | 4 | 4 | 4 | 5 | 4 | 4 | 4 | 4 |
| Sinapis | 2 | 2 | 2 | 2 | 3 | 3 | 3 | 3 | 2 | 2 | 2 | 2 |
| Stellaria | 2 | 2 | 2 | 2 | 2 | 3 | 4 | 4 | 2 | 2 | 2 | 2 |
| Chrysanthe. leuc. | 1 | 1 | 1 | 1 | 3 | 3 | 3 | 3 | 2 | 2 | 2 | 2 |
| Galium aparine | 7 | 7 | 7 | 7 | 3 | 4 | 6 | 6 | 1 | 1 | 1 | 1 |
| Viola tricolor | 2 | 3 | 3 | 3 | 6 | 6 | 6 | 6 | 2 | 2 | 2 | 2 |
| Veronica Sp. | 2 | 2 | 2 | 2 | 6 | 6 | 6 | 6 | 2 | 2 | 2 | 2 |

Beispiel 7

Nachweis der Keimhemmung an Lagerkartoffeln.

Eine Anzahl im Handel erhältliche Kartoffeln der Sorte «Urgenta» ohne Keime werden gewaschen und abgetrocknet. Danach werden die Kartoffeln für jeweils eine Minute in Wirkstoffemulsionen verschiedener Konzentration getaucht, in Kunststoffschalen auf Filterpapier ausgelegt und bei Temperaturen von 14° und 21°C im Dunkeln bei 50% relativer Luftfeuchtigkeit gehalten. Die Auswertung erfolgte 34 Tage nach der Applikation. Die Bewertung wurde nach folgendem Massstab vorgenommen:

1: Keine Keimbildung (wie bei Applikation)
5: ca. 50% Hemmung der Keimbildung
9: Keimbildung wie bei der unbehandelten Kontrolle.

Gleichzeitig wird der Gewichtsverlust der Knollen in % und das Gewicht der Keime im Vergleich zur unbehandelten Kontrolle ermittelt.

**Versuchergebnisse:** Keimhemmung bei Lagerkartoffeln

| Verb. Nr. | Konz. Tauch-brühe (ppm) | Lagerort 14°C<br>Keime<br>Keim-bildung | <br><br>Gewicht (g) | <br><br>Gewicht (% Kontrolle) | <br>Knollen<br>Gewichts-verlust (%) | Lagerort 21°C<br>Keime<br>Keim-bildung | <br><br>Gewicht (g) | <br><br>Gewicht (% Kontrolle) | <br>Knollen<br>Gewichts-verlust (%) |
|---|---|---|---|---|---|---|---|---|---|
| Kontr. | 0 | 9 | 52,8 | 100 | 10,4 | 9 | 50,9 | 100 | 12,2 |
| 1 | 1000 | 1 | 0 | 0 | 5,5 | 3 | 3,0 | 5,9 | 8,4 |
| 3 | 1000 | 3 | 4,8 | 9,1 | 5,4 | 7 | 39,4 | 77,4 | 11,3 |

Beispiel 8

Nachweis der Wuchshemmung bei tropischen Bodenbedeckern-Leguminosen (cover crops).

Die Versuchspflanzen (centrosema plumieri und centrosema pubescens) werden bis zum ausgewachsenen Stadium herangezogen und bis auf eine Höhe von 60 cm zurückgeschnitten. Nach 7 Tagen wird der Wirkstoff als wässrige Emulsion gespritzt. Die Versuchspflanzen werden bei 70% relativer Luftfeuchtigkeit und 6000 lux Kunstlicht, pro Tag 14 Stunden bei Temperaturen von 27° bei Tag und 21°C bei Nacht gehalten. 4 Wochen nach der Applikation wird der Versuch ausgewertet. Es werden dabei

a) der Neuzuwachs in % zur Kontrolle abgeschätzt
b) der Neuzuwachs in % zur Kontrolle gewogen

und

c) die Phytotoxizität nach der folgenden Linearskala bewertet:

1: Pflanze abgestorben;
9: Pflanze nicht geschädigt.

**Versuchsergebnisse:** Wachstumshemmung bei cover crops

| Pflanze | Verb. Nr. | Aufwandmenge gAS/ha | Neuzuwachs visuell % Kontrolle | Neuzuwachs gewogen g | Neuzuwachs gewogen % Kontrolle | Phytoxizität |
|---|---|---|---|---|---|---|
| Centrosema pubescens | 1 | 10 | 0 | 2 | 4 | 8 |
| | 1 | 30 | 10 | 9 | 18 | 8 |
| | 1 | 100 | 0 | 2 | 4 | 8 |
| | 3 | 10 | 0 | 1 | 2 | 9 |
| | 3 | 30 | 0 | 2 | 4 | 9 |
| | 3 | 100 | 0 | 0 | 0 | 9 |
| Centrosema plumieri | 1 | 10 | 20 | 31 | 50 | 9 |
| | 1 | 30 | 0 | 0 | 0 | 8 |
| | 1 | 100 | 0 | 4 | 6 | 8 |
| | 3 | 10 | 30 | 29 | 47 | 9 |
| | 3 | 30 | 10 | 26 | 42 | 9 |
| | 3 | 100 | 0 | 6 | 10 | 9 |

**Patentansprüche** für die Vertragsstaaten BE, CH, LI, FR, GB, IT, NL, SE

1. N-(2-substituierte Phenylsulfonyl)-N'-triazinylharnstoffe der allgemeinen Formel I

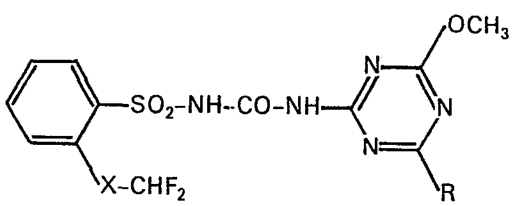

(I),

worin X Sauerstoff und R Methyl oder Methoxy bedeuten, und ausserdem X für Schwefel stehen kann, wenn R Methyl bedeutet, sowie die Salze dieser Verbindungen.

2. N-(2-Difluormethoxyphenylsulfonyl)-N'-(4,6-dimethoxy-1,3,5-triazin-2-yl)-harnstoff gemäss Anspruch 1.

3. N-(2-Difluormethoxyphenylsulfonyl)-N'-(4-methyl-6-methoxy-1,3,5-triazin-2-yl)-harnstoff gemäss Anspruch 1.

4. N-(2-Difluormethylthiophenylsulfonyl)-N'-(4-methyl-6-methoxy-1,3,5-triazin-2-yl)-harnstoff gemäss Anspruch 1.

5. Verfahren zur Herstellung der Verbindung der Formel I, Anspruch 1, dadurch gekennzeichnet, das man ein Phenylsulfonylisocyanat der Formel IV

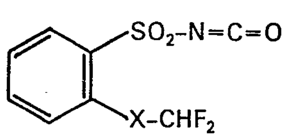

(IV),

worin X die unter Formel I gegebene Bedeutung hat, gegebenenfalls in Gegenwart einer Base, mit einem Amin der Formel V

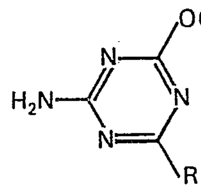

(V),

worin R die unter Formel I gegebene Bedeutung hat, umsetzt und gegebenenfalls in ihre Salze überführt.

6. Verfahren zur Herstellung der Verbindungen der Formel I, Anspruch 1, dadurch gekennzeichnet, dass man N-Phenylsulfonylcarbamat der Formel VII

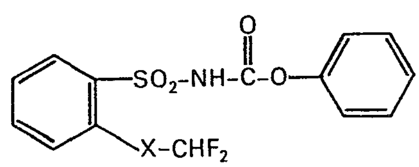

(VII),

worin X die unter Formel I gegebene Bedeutung hat, mit einem Amin der oben angegebenen Formel V umsetzt und gegebenenfalls in ihre Salze überführt.

7. Verfahren zur Herstellung von basischen Additionssalzen der Formel I gemäss einem der Ansprüche 5 bis 6, dadurch gekennzeichnet, dass man einen Sulfonylharnstoff der Formel I mit einem Amin, einem Alkalimetall- oder Erdalkalimetallhydroxid oder einer quaternären Ammoniumbase umsetzt.

8. Ein herbizides und den Pflanzenwuchs hemmendes Mittel, dadurch gekennzeichnet, dass es neben Träger- und/oder anderen Zuschlagstoffen als Wirkstoff mindestens einen N-Phenylsulfonyl-N'-triazinyl-harnstoff der Formel I, Anspruch 1, enthält.

9. Die Verwendung der N-Phenylsulfonyl-N'-triazinyl-harnstoffe der Formel I, Anspruch 1, oder sie enthaltender Mittel zur Bekämpfung unerwünschten Pflanzenwachstums.

10. Die Verwendung der N-Phenylsulfonyl-N'-triazinyl-harnstoffe der Formel I, Anspruch 1, oder sie enthaltender Mittel zur Hemmung des Pflanzenwachstums.

11. Die Verwendung der N-Phenylsulfonyl-N'-triazinyl-harnstoffe der Formel I, oder sie enthaltender Mittel, gemäss Anspruch 9, zur selektiven pre- oder postemergenten Bekämpfung von Unkräutern in Nutzpflanzenkulturen.

12. Die Verwendung der N-Phenylsulfonyl-N'-triazinyl-harnstoffe der Formel I, oder sie enthaltender Mittel, gemäss Anspruch 10, zur Unterdrückung des Pflanzenwachstums über das 2-Blattstadium hinaus, dadurch gekennzeichnet, dass die Wirkstoffe pre-emergent angewendet werden.

13. Die Verwendung der Verbindungen der Formel I oder sie enthaltender Mittel gemäss Anspruch 10 zur Wuchshemmung von Bodenbedeker-Leguminosen.

14. Die Verwendung der Verbindungen der Formel I oder sie enthaltender Mittel gemäss Anspruch 10 zur Keimhemmung von Lagerkartoffeln.

**Patentansprüche** für den Vertragsstaat AT

1. Ein herbizides und den Pflanzenwuchs hemmendes Mittel, dadurch gekennzeichnet, dass es neben Träger- und/oder anderen Zuschlagstoffen als Wirkstoff mindestens einen N-(2-substituierte Phenylsulfonyl)-N'-triazinylharnstoff der allgemeinen Formel

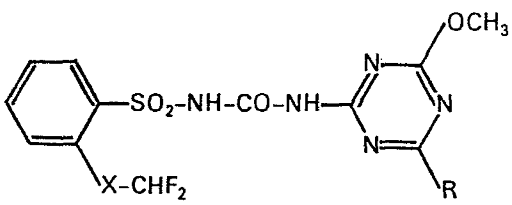

(I),

worin X Sauerstoff und R Methyl oder Methoxy bedeuten und ausserdem X für Schwefel stehen kann, wenn R Methyl bedeutet, oder ein Salz dieser Verbindungen enthält.

2. Mittel gemäss Anspruch 1, dadurch gekennzeichnet, dass es als Wirkstoff N-(2-Difluormethoxyphenylsulfonyl)-N'-(4,6-dimethoxy-1,3,5-triazin-2-yl)-harnstoff enthält.

3. Mittel gemäss Anspruch 1, dadurch gekennzeichnet, dass es als Wirkstoff N-(2-Difluormethoxyphenylsulfonyl)-N'-(4-methyl-6-methoxy-1,3,5-triazin-2-yl)-harnstoff enthält.

4. Mittel gemäss Anspruch 1, dadurch gekennzeichnet, dass es als Wirkstoff N-(2-Difluormethylthiophenylsulfonyl)-N'-(4-methyl-6-methoxy-1,3,5-triazin-2-yl)-harnstoff enthält.

5. Verfahren zur Herstellung der Verbindungen der Formel I nach Anspruch 1, dadurch gekennzeichnet, dass man ein Phenylsulfonylisocyanat der Formel IV

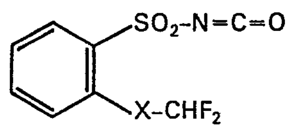

(IV),

worin X die unter Formel I gegebene Bedeutung hat, gegebenenfalls in Gegenwart einer Base, mit einem Amin der Formel V

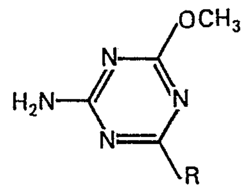

(V),

worin R die unter Formel I gegebene Bedeutung hat, umsetzt und gegebenenfalls in ihre Salze überführt.

6. Verfahren zur Herstellung der Verbindungen der Formel I, nach Anspruch 1, dadurch gekennzeichnet, dass man N-Phenylsulfonylcarbamat der Formel VII

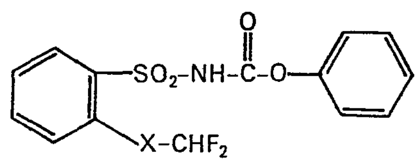

(VII),

worin X die unter Formel I gegebene Bedeutung hat, mit einem Amin der oben angegebenen Formel V umsetzt und gegebenenfalls in ihre Salze überführt.

7. Verfahren zur Herstellung von basischen Additionssalzen der Formel I gemäss einem der Ansprüche 5 bis 6, dadurch gekennzeichnet, dass man einen Sulfonylharnstoff der Formel I mit einem Amin, einem Alkalimetall- oder Erdalkalimetallhydroxid oder einer quaternären Ammoniumbase umsetzt.

8. Die Verwendung der N-Phenylsulfonyl-N'-triazinyl-harnstoffe der Formel I, nach Anspruch 1, oder sie enthaltender Mittel zur Bekämpfung unerwünschten Pflanzenwachstums.

9. Die Verwendung der N-Phenylsulfonyl-N'-triazinyl-harnstoffe der Formel I, nach Anspruch 1, oder sie enthaltender Mittel zur Hemmung des Pflanzenwachstums.

10. Die Verwendung der N-Phenylsulfonyl-N'-triazinyl-harnstoffe der Formel I, oder sie enthaltender Mittel, gemäss Anspruch 8, zur selektiven pre- oder postemergenten Bekämpfung von Unkräutern in Nutzpflanzenkulturen.

11. Die Verwendung der N-Phenylsulfonyl-N'-triazinyl-harnstoffe der Formel I, oder sie enthaltender Mittel, gemäss Anspruch 9, zur Unterdrückung des Pflanzenwachstums über das 2-Blattstadium hinaus, dadurch gekennzeichnet, dass die Wirkstoffe pre-emergent angewendet werden.

12. Die Verwendung der Verbindungen der Formel I oder sie enthaltender Mittel gemäss Anspruch 9 zur Wuchshemmung von Bodenbedeker-Leguminosen.

13. Die Verwendung der Verbindungen der Formel I oder sie enthaltender Mittel gemäss Anspruch 9 zur Keimhemmung von Lagerkartoffeln.

**Claims** for the Contracting States BE, CH, LI, FR, GB, IT, NL, SE

1. A N-(2-substituted phenylsulfonyl)-N′-triazinylurea of the general formula I

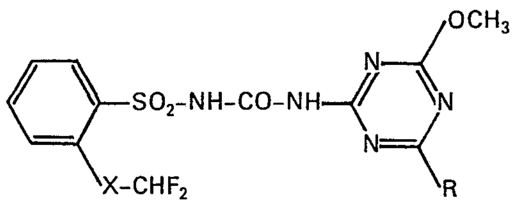

(I),

wherein X is oxygen and R is methyl or methoxy, whilst X can also be sulfur if R is methyl, or a salt thereof.

2. N-(2-Difluoromethoxyphenylsulfonyl)-N′-(4,6-dimethoxy-1,3,5-triazin-2-yl)urea according to claim 1.

3. N-(2-Difluoromethoxyphenylsulfonyl)-N′-(4-methyl-6-methoxy-1,3,5-triazin-2-yl)urea according to claim 1

4. N-(2-Difluoromethylthiophenylsulfonyl)-N′-(4-methyl-6-methoxy-1,3,5-triazin-2-yl)urea according to claim 1.

5. A process for the preparation of a compound of the formula I according to claim 1, which comprises reacting a phenylsulfonylisocyanate of the formula IV

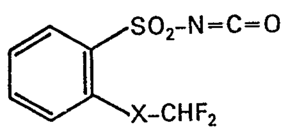

(IV),

wherein X is as defined for formula I, optionally in the presence of a base, with an amine of the formula V

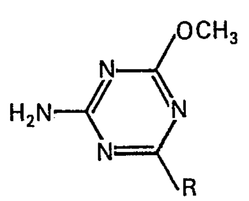

(V),

wherein R is as defined for formula I, and, if desired, converting the reaction product into a salt.

6. A process for the preparation of a compound of the formula I according to claim 1, which comprises reacting a N-phenylsulfonylcarbamate of the formula VII

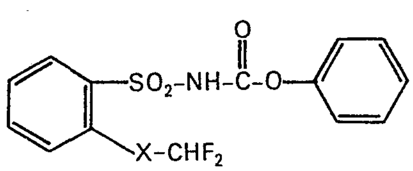

(VII),

wherein X is as defined for formula I, with an amine of the formula V above and, if desired, converting the reaction product into a salt.

7. A process for the preparation of a basic addition salt of the formula I according to either claim 5 or claim 6, which comprises reacting a sulfonylurea of the formula I with an amine, an alkali metal hydroxide or alkaline earth metal hydroxide or with a quaternary ammonium base.

8. A herbicidal and plant growth-inhibiting composition which contains at least one N-phenylsulfonyl-N′-triazinylurea of the formula I according to claim 1, together with carriers and/or other adjuvants.

9. A method of controlling undesired plant growth, which method comprises applying an effective amount of an N-phenylsulfonyl-N′-triazinylurea of the formula I according to claim 1 or of a composition containing such a compound.

10. A method of inhibiting plant growth, which method comprises applying an effective amount of an N-phenylsulfonyl-N′-triazinylurea of the formula I according to claim 1 or of a composition containing such a compound.

11. A method according to claim 9 of selectively controlling weeds pre- and postemergence in crops of useful plants, which comprises applying an effective amount of an N-phenylsulfonyl-N′-triazinylurea of the formula I or of a composition containing such a compound.

12. A method according to claim 10 of suppressing plant growth beyond the two-leaf stage, which comprises applying preemergence an N-phenylsulfonyl-N′-triazinylurea of the formula I or of a composition containing such a compound.

13. A method according to claim 10 of inhibiting the growth of cover crops of the species leguminosae, which comprises applying an effective amount of a compound of the formula I or of a composition containing such a compound.

14. A method according to claim 10 of inhibiting the growth of shoots of stored potatoes, which comprises applying an effective amount of a compound of the formula I or of a composition containing such a compound.

**Claims** for the Contracting State AT

1. A herbicidal and plant growth-inhibiting composition which contains at least one N-(2-substituted phenylsulfonyl)-N′-triazinylurea of the general formula I

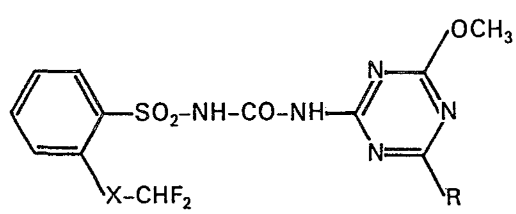

(I),

wherein X is oxygen and R is methyl or methoxy, whilst X can also be sulfur if R is methyl, or a salt thereof, together with carriers and/or other adjuvants.

2. A composition according to claim 1, which contains N-(2-difluoromethoxyphenylsulfonyl)-N'-(4,6-dimethoxy-1,3,5-triazin-2-yl)urea as active component.

3. A composition according to claim 1, which contains N-(2-difluoromethoxyphenylsulfonyl)-N'-(4-methyl-6-methoxy-1,3,5-triazin-2-yl)urea as active component.

4. A composition according to claim 1, which contains N-(2-difluoromethylthiophenylsulfonyl)-N'-(4-methyl-6-methoxy-1,2,5-triazin-2-yl)urea as active component.

5. A process for the preparation of a compound of the formula I according to claim 1, which comprises reacting a phenylsulfonylisocyanate or phenylsulfonylisothiocyanate of the formula IV

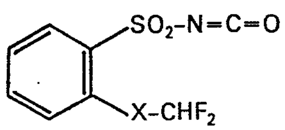 (IV),

wherein X is as defined for formula I, optionally in the presence of a base, with an amine of the formula V

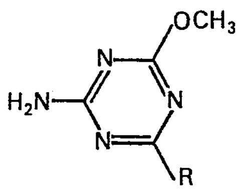 (V),

wherein R is as defined for formula I, and, if desired, converting the reaction product into a salt.

6. A process for the preparation of a compound of the formula I according to claim 1, which comprises reacting a N-phenylsulfonylcarbamate of the formula VII

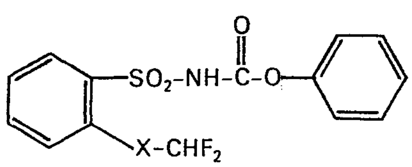 (VII),

wherein X is as defined for formula I, with an amine of the formula V above and, if desired, converting the reaction product into a salt.

7. A process for the preparation of a basic addition salt of the formula I according to either claim 5 or claim 6, which comprises reacting a sulfonylurea of the formula I with an amine, an alkali metal hydroxide or alkaline earth metal hydroxide or with a quaternary ammonium base.

8. A method of controlling undesired plant growth, which method comprises applying an effective amount of an N-phenylsulfonyl-N'-triazinylurea of the formula I according to claim 1 or of a composition containing such a compound.

9. A method of inhibiting plant growth, which method comprises applying an effective amount of an N-phenylsulfonyl-N'-triazinylurea of the formula I according to claim 1 or of a composition containing such a compound.

10. A method according to claim 9 of selectively controlling weeds pre- and postemergence in crops of useful plants, which comprises applying an effective amount of an N-phenylsulfonyl-N'-triazinylurea of the formula I or of a composition containing such a compound.

11. A method according to claim 10 of suppressing plant growth beyond the two-leaf stage, which comprises applying preemergence an N-phenylsulfonyl-N'-triazinylurea of the formula I or of a composition containing such a compound.

12. A method according to claim 10 of inhibiting the growth of cover crops of the species leguminosae, which comprises applying an effective amount of a compound of the formula I or of a composition containing such a compound.

13. A method according to claim 10 of inhibiting the growth of shoots of stored potatoes, which comprises applying an effective amount of a compound of the formula I or of a composition containing such a compound.

**Revendications** pour les Etats contractants BE, CH, LI, FR, GB, IT, NL, SE

1. N-(phénylsulfonyle-2-substitué)-N'-triazinylurées de formule générale I

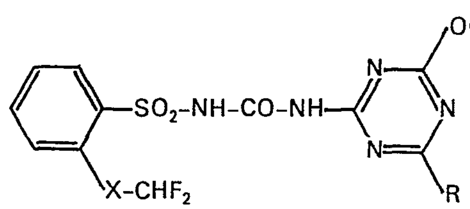 (I),

dans laquelle X représente l'oxygène et R représente un groupe méthyle ou méthoxy, X pouvant en outre représenter le soufre lorsque R représente le groupe méthyle, et les sels de ces composés.

2. La N-(2-difluorométhoxyphénylsulfonyl)-N'-(4,6-diméthoxy-1,3,5-triazine-2-yl)-urée selon la revendication 1.

3. La N-(2-difluorométhoxyphénylsulfonyl)-N'-(4-méthyl-6-méthoxy-1,3,5-triazine-2-yl)-urée selon la revendication 1.

4. La N-(2-difluorométhylthiophénylsulfonyl)-N'-(4-méthyl-6-méthoxy-1,3,5-triazine-2-yl)-urée selon la revendication 1.

5. Procédé de préparation des composés de formule I, revendication 1, caractérisé en ce que l'on fait réagir un phénylsulfonylisocyanate de formule IV

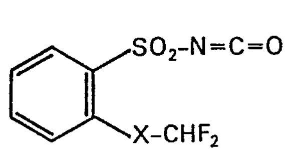 (IV),

dans laquelle X a la signification indiquée en référence à la formule I, éventuellement en présence d'une base, avec une amine de formule V

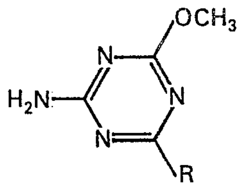

(V),

dans laquelle R a la signification indiquée en référence à la formule I, et on convertit éventuellement en sels.

6. Procédé de préparation des composés de formule I, revendication 1, caractérisé en ce que l'on fait réagir un N-phénylsulfonylcarbamate de formule VII

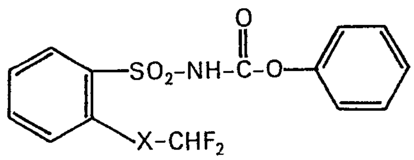

(VII),

dans laquelle X a la signification indiquée en référence à la formule I, avec une amine de formule V ci-dessus, et on convertit éventuellement en sels.

7. Procédé de préparation de sels formés par addition avec une base, de formule I selon les revendications 5 et 6, caractérisé en ce que l'on fait réagir une sulfonylurée de formule I avec une amine, un hydroxyde de métal alcalin ou alcalino-terreux ou une base d'ammonium quaternaire.

8. Un produit herbicide et inhibiteur de la croissance des végétaux, caractérisé en ce qu'il contient en tant que substance active, avec des véhicules et/ou d'autres additifs, au moins une N-phénylsulfonyl-N'-triazinylurée de formule I, revendication 1.

9. L'utilisation des N-phénylsulfonyl-N'-triazinyl-urées de formule I, revendication 1, ou de produits en contenant, pour la lutte contre les croissances indésirables de végétaux.

10. L'utilisation des N-phénylsulfonyl-N'-triazinyl-urées de formule I, revendication 1, ou de produits en contenant, pour l'inhibition de la croissance des végétaux.

11. L'utilisation des N-phénylsulfonyl-N'-triazinyl-urées de formule I ou de produits en contenant, selon la revendication 9, pour la lutte sélective en pré-levée ou post-levée contre les mauvaises herbes dans les cultures de végétaux utiles.

12. L'utilisation des N-phénylsulfonyl-N'-triazinyl-urées de formule I ou de produits en contenant, selon la revendication 10, pour inhiber la croissance des végétaux au-delà du stade 2 feuilles, caractérisée en ce que l'on applique les substances actives en pré-levée.

13. L'utilisation des composés de formule I ou de produits en contenant, selon la revendication 10, pour inhiber la croissance des légumineuses de couvertures de sol.

14. L'utilisation des composés de formule I ou de produits en contenant, selon la revendication 10, pour l'inhibition de la germination des pommes de terre stockées.

**Revendications** pour l'Etat contractant AT

1. Un produit herbicide et inhibiteur de la croissance des végétaux, caractérisé en ce qu'il contient en tant que substance active, avec des véhicules et/ou d'autres additifs au moins une N-(phénylsulfonyle-2-substitué)-N'-triazinylurée de formule générale

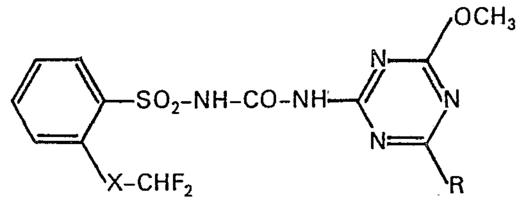

(I),

dans laquelle X représente l'oxygène et R représente un groupe méthyle ou méthoxy, X pouvant en outre représenter le soufre lorsque R représente le groupe méthyle, et les sels de ces composés.

2. Produit selon la revendication 1 caractérisé en ce qu'il contient comme substance active N-(2-difluorométhoxyphénylsulfonyl)-N'-(4,6-diméthoxy-1,3,5-triazine-2-yl)-urée.

3. Produit selon la revendication 1 caractérisé en ce qu'il contient comme substance active N-(2-difluorométhoxyphénylsulfonyl)-N'-(4-méthyl-6-méthoxy-1,3,5-triazine-2-yl)-urée.

4. Produit selon la revendication 1 caractérisé en ce qu'il contient comme substance active N-(2-difluorométhylthiophénylsulfonyl)-N'-(4-méthyl-6-méthoxy-1,3,5-triazine-2-yl)-urée.

5. Procédé de préparation des composés de formule I, revendication 1, caractérisé en ce que l'on fait réagir un phénylsulfonylisocyanate ou phénylsulfonylisothiocyanate de formule IV

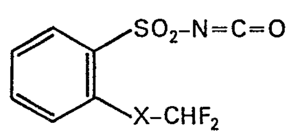

(IV),

dans laquelle X a la signification indiquée en référence à la formule I, éventuellement en présence d'une base, avec une amine de formule V

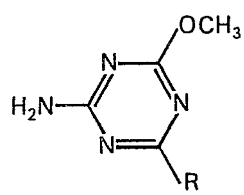

(V),

dans laquelle R a la signification indiquée en référence à la formule I, et on convertit éventuellement en sels.

6. Procédé de préparation des composés de formule I, revendication 1, caractérisé en ce que

l'on fait réagir un N-phénylsulfonylcarbamate de formule VII

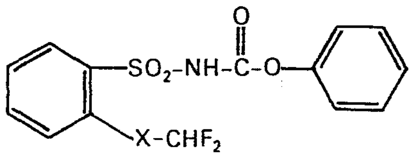

(VII),

dans laquelle X a la signification indiquée en référence à la formule I, avec une amine de formule V ci-dessus, et on convertit éventuellement en sels.

7. Procédé de préparation de sels formés par addition avec une base, de formule I selon les revendications 5 et 6, caractérisé en ce que l'on fait réagir une sulfonylurée de formule I avec une amine, un hydroxyde de métal alcalin ou alcalino-terreux ou une base d'ammonium quaternaire.

8. L'utilisation des N-phénylsulfonyl-N'-triazinyl-urées de formule I, revendication 1, ou de produits en contenant, pour la lutte contre les croissances indésirables de végétaux.

9. L'utilisation des N-phénylsulfonyl-N'-triazinyl-urées de formule I, revendication 1, ou de produits en contenant, pour l'inhibition de la croissance des végétaux.

10. L'utilisation des N-phénylsulfonyl-N'-triazinyl-urées de formule I ou de produits en contenant, selon la revendication 9, pour la lutte sélective en pré-levée ou post-levée contre les mauvaises herbes dans les cultures de végétaux utiles.

11. L'utilisation des N-phénylsulfonyl-N'-triazinyl-urées de formule I ou de produits en contenant, selon la revendication 10, pour inhiber la croissance des végétaux au-delà du stade 2 feuilles, caractérisée en ce que l'on applique les substances actives en pré-levée.

12. L'utilisation des composés de formule I ou de produits en contenant, selon la revendication 10, pour inhiber la croissance des légumineuses de couvertures de sol.

13. L'utilisation des composés de formule I ou de produits en contenant, selon la revendication 10, pour l'inhibition de la germination des pommes de terre stockées.